# EUROPEAN PATENT SPECIFICATION

(11) **EP 2 528 532 B1**
(45) Date of publication and mention of the grant of the patent: **14.01.2015**
(21) Application number: 11737810.9
(22) Date of filing: 31.01.2011
(51) Int. Cl.: A61B 19/00, A61N 5/10, A61B 6/04, A61G 13/12

(54) **LOCAL ADJUSTMENT DEVICE FOR RADIOTHERAPY**
LOKALE EINSTELLUNGSVORRICHTUNG FÜR RADIOTHERAPIE
DISPOSITIF DE RÉGLAGE LOCAL POUR RADIOTHÉRAPIE

(30) Priority: 29.01.2010 US 696595
(43) Date of publication of application: 05.12.2012
(73) Proprietor: William Beaumont Hospital, Royal Oak, MI 48072-6769 (US)
(72) Inventor: YAN, Di, Auburn Hills, MI 48326 (US)
(74) Representative: Solf, Alexander
(86) International application number: PCT/US2011/023149
(87) International publication number: WO 2011/094674

(56) References cited:
- EP-A1- 2 141 009
- WO-A1-93/21867
- WO-A1-98/17177
- WO-A1-98/41126
- US-A- 4 034 748
- US-A- 4 034 748
- US-A- 4 297 994
- US-A- 4 543 947
- US-A1- 2002 103 520
- US-A1- 2005 033 207
- US-A1- 2005 251 914
- US-A1- 2009 070 938
- US-B1- 7 146 664
- US-B2- 6 656 143
- US-B2- 7 309 321

## Description

### BACKGROUND OF THE INVENTION

This invention relates to a local adjustment device for radiotherapy. Present procedures for head and neck radiotherapy require the molding of a cradle to position the patient's head. The patient wears a mask which clamps the head in position against the formed cradle. The forming of a mold dedicated to each particular patient is costly and requires a significant amount of time. Moreover, changes in the patient anatomy can occur during the course of treatment, requiring recasting of the mold.

### BRIEF SUMMARY OF THE INVENTION

The present invention provides a means to position an anatomical portion of a patient during radiation therapy treatment.

The present invention provides a cradle for positioning an anatomical portion of a patient during radiation therapy treatment. The cradle comprises a plurality of expandable fluid chambers, each of the plurality of fluid chambers being configured to exert a force against one of a plurality of surfaces of the anatomical portion; and a pressure regulator configured to adjust the pressure of each of the fluid chambers independently of every other fluid chamber by supplying fluid to or withdrawing fluid from each of the fluid chambers.

The present invention provides a system for positioning an anatomical portion of a patient during radiation therapy treatment. The system comprises a cradle and a control unit. The cradle comprises a plurality of expandable fluid chambers, each of the plurality of fluid chambers being configured to exert a force against one of a plurality of surfaces of the anatomical portion, at least two of the plurality of fluid chambers exerting substantially opposing forces against the anatomical portion, such that the anatomical portion is immobilized in the cradle; and a pressure regulator configured to adjust the pressure of each of the fluid chambers independently of every other fluid chamber by supplying fluid to or withdrawing fluid from each of the fluid chambers. The control unit is configured to instruct the pressure regulator to adjust the pressure of each of the fluid chambers, such that the anatomical portion is held in a desired position.

The invention is defined in the claims. Other embodiments are presented for illustrative purposes only.

### BRIEF DESCRIPTION OF THE DRAWINGS

In the accompanying drawings, the components are not necessarily drawn to scale, emphasis instead being placed upon illustrating the principles of the invention. Moreover, like reference numerals designate corresponding parts throughout the views. In the drawings:
FIG. 1 schematically shows an embodiment of a radiation therapy system that employs a dose tracking and feedback process and a possible workflow for auto-construction, estimation and evaluation of cumulative treatment dose, and patient anatomy and dose feedback for adaptive planning optimization in accordance with the present invention;
FIGs. 2a-2e show an embodiment of an onboard imaging system to be used with the radiation therapy system of FIG. 1 for performing dose tracking and feedback in accordance with the invention;
FIG. 3 is a perspective view of a cradle for positioning a patient's head during treatment in accordance with the invention;
FIG.4 is a top view of a system for positioning a patient's head during treatment in accordance with the invention;
FIG. 5 is a side view of the system of FIG. 4; and
FIG. 6 is a perspective view of another embodiment of a cradle for positioning a patient's head during treatment in accordance with the invention.

### DETAILED DESCRIPTION OF THE INVENTION

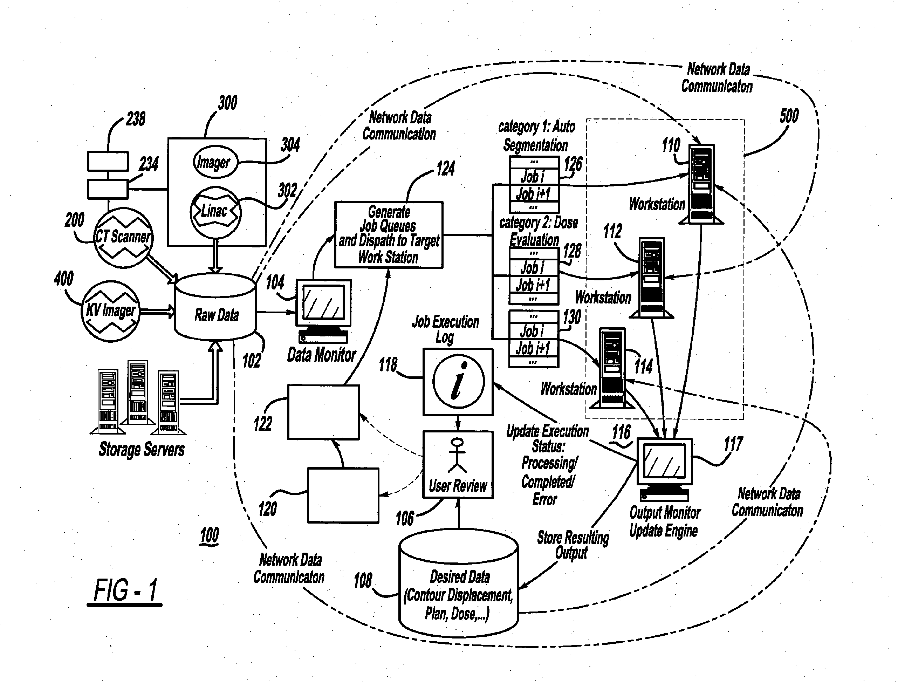

Referring now to FIG. 1, generally illustrated therein is a schematic view of a volumetric image guided adaptive radiotherapy system, such as cone-beam computerized tomography (CBCT) image guided adaptive radiotherapy (IGART) system 100, and a corresponding workflow sequence for auto-construction and evaluation of daily cumulative treatment dose. As shown in FIG. 1, the CBCT IGART system 100 includes a number of major systems: 1) a three-dimensional volumetric imaging system, such as an x-ray cone-beam computed tomography system 200, 2) a megavoltage (MeV) system 300 that includes a radiation therapy source, such as a linear accelerator 302, and an imager 304, 3) a kilovoltage (kV) portal imager processor/software system 400, and 4) a treatment dose tracking and feedback system 500, each of which are discussed below.

Mechanical operation of a cone-beam computed tomography system 200 is similar to that of a conventional computed tomography system, with the exception that an entire volumetric image is acquired through less than two rotations (preferably one rotation) of the source and detector. This is made possible by the use of a two-dimensional (2-D) detector, as opposed to the one-dimensional (1-D) detectors used in conventional computed tomography.

An example of a known cone-beam computed tomography imaging system is described in U.S. Patent No. 6,842,502 (the '502 patent). The '502 patent describes an embodiment of a cone-beam computed tomography imaging system that includes a kilovoltage x-ray tube and a flat panel imager having an array of amorphous silicon detectors. As a patient lies upon a treatment table, the x-ray tube and flat panel imager rotate about the patient in unison so as to take a plurality of images.

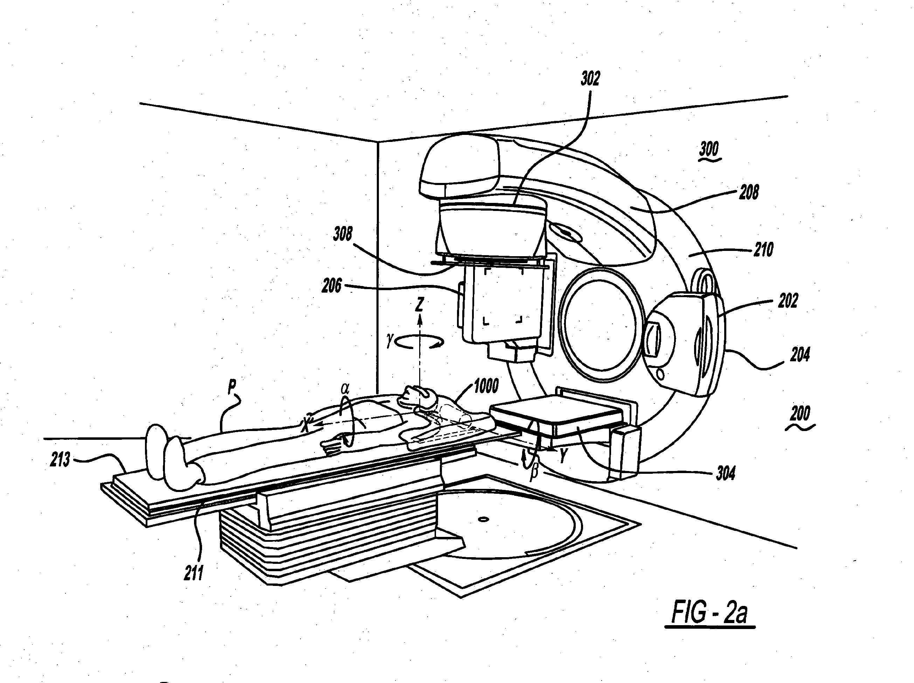

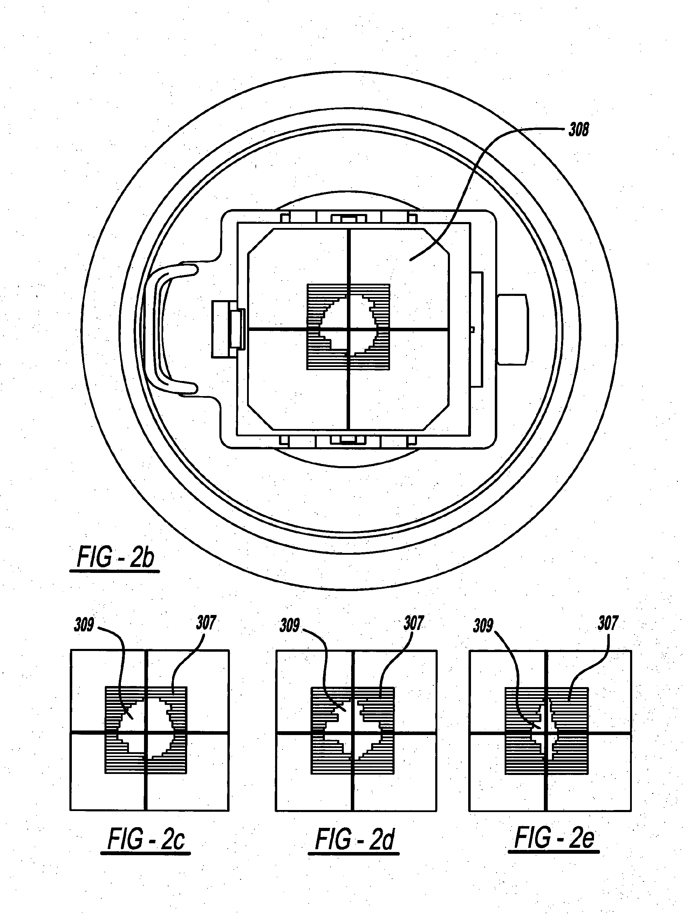

A volumetric imaging system is illustrated in FIG. 2a. In particular, FIG. 2a shows an embodiment of a wall-mounted cone-beam computed tomography system 200 and MeV portal imaging system 300 sold under the trade name Synergy by Elekta of Crawley, the United Kingdom. Such systems 200 and 300 are described in U.S. Patent No. 7,760,849.

The cone-beam computed tomography system 200 includes an x-ray source, such as x-ray tube 202, a rotary collimator 204 and a flat-panel imager/detector 206 mounted on a gantry 208. As shown in FIG. 2a, the CBCT system 200 can be mounted to the face of a flat, circular, rotatable drum 210 of the gantry 208 of a medical linear accelerator 302, where the x-ray beam produced by the x-ray tube 202 is approximately orthogonal to the treatment beam produced by the radiation therapy source 302.

Note that the detector 206 can be composed of a two-dimensional array of semiconductor sensors that may be each made of amorphous silicon (*a*-Si:H) and thin-firm transistors. The analog signal from each sensor is integrated and digitized. The digital values are transferred to the data storage server 102.

After the fan beams from collimator 204 traverse the width of a patient P and impinge on the entire detector 206 in the manner described above, computer 234 (shown in FIG. 1) instructs the drum 210 to rotate causing the x-ray source 202, the collimator 204 and the detector 206 rotate about the patient P to another position so that the scanning process described above can be repeated and another two-dimensional projection is generated. The above rotation of the x-ray source 202, collimator 204 and detector 206 is continued until a sufficient number of two-dimensional images are acquired for forming a cone-beam computed tomography image. Less than two rotations should be needed for this purpose (images formed from a rotation of less than 360° can be formed as well). The two-dimensional projections from each position are combined in the computer 234 to generate a three-dimensional image to be shown on display 238 in a manner similar to that of the cone-beam computed tomography systems described previously.

As shown in FIG. 2a, the MeV imaging system 300 includes a separate radiation therapy source 302 and a detector/imager 304 that are separately mounted to the rotating drum 210. The source 302 operates at a power level higher than that of x-ray tube 202 so as to allow for treatment of a target volume in a patient lying on a movable table 211 (movable in x, y and z-direction via computer 234). The source 302 generates a beam of x-rays or particles, such as photons, protons or electrons, which have an energy ranging from about 4 MeV to about 25 MeV. The beam from source 302, of whatever type, is referred to here as radiation.

As mentioned above, the radiation from source 302 is used to treat a specific area of interest of a patient, such as a tumor. Prior to arriving at the area of interest, the radiation beam is shaped by adjusting multiple leafs 307 (FIGS. 2b-e) to have a particular cross-sectional area 309 via a multi-leaf collimator 308. The cross-sectional area 309 is chosen so that the beam interacts with the area of interest to be treated and not areas of the patient that are healthy. The radiation penetrating through the area of interest can be imaged via imager 304 in a well known manner.

As shown in FIG. 1, the treatment dose tracking and feedback system 500 includes a workstation or data server 110 that includes processors dedicated to perform a segmentation/registration process on a three-dimensional, volumetric image of a patient received from server 102 that was generated by cone-beam computed tomography system 200. The workstation 110 is able to identify and register each volume of image data within each volumetric image. Such identification and registration allows for the same volume of image data to be tracked in position from one therapy session to another therapy session.

The treatment dose tracking and feedback system 500 further includes a workstation or data server 112 that includes processors dedicated to perform a treatment dose construction process based on 1) the segmentation/registration process performed by workstation 110 and 2) parameters of the beam of radiation emitted from the source 302 as it impinges on the patient that are measured and stored in server 102, such as angular position, beam energy and cross-sectional shape of the beam, in accordance with a reference plan. Such parameters can be in the form of the angular position of the gantry 208, the angular orientation of the collimator 308, the positions of the leaves of the multi-leaf collimator 308, position of the table 211 and energy of the radiation beam. Once the position and shape of a subvolume of image data is known, the treatment dosage received by that very same subvolume can be determined/constructed based on the above mentioned parameters of the beam of radiation emitted from the source 302 as it impinges on the patient. Such a determination is made for each of the subvolumes of image data for each of the volumetric images generated by system 200.

The treatment dose tracking and feedback system 500 further includes a workstation or data server 114 that includes processors dedicated to perform an adaptive planning process that can either 1) adjust the radiation therapy treatment for the particular day in a real-time manner based on off-line and on-line information or 2) adjust a radiation therapy treatment plan in a non-real-time manner based on off-line information. The adjustment is based on how the dose calculated by the workstation 112 differs from dose preferred by the treatment plan. Note that the term "real-time" refers to the time period when the radiation therapy source is activated and treating the patient. The term "on-line" regards when a patient is on the treatment table and "off-line" refers to when the patient is off the treatment table.

In summary, the treatment dose tracking and feedback system 500 can perform real time treatment dose construction and 4D adaptive planning based on volumetric image information and therapy beam parameters that are measured in a real time manner during a therapy session. The system 500 can also perform adaptive planning in a non-real-time manner. Note that in an alternative embodiment, the workstations 110, 112 and 114 can be combined into a single workstation wherein the processes associated with workstations 110, 112 and 114 are performed by one or more processors. Note that the real time treatment dose construction determined by workstation 112 and the 4D adaptive planning determined by workstation 114 can be displayed on a monitor 117 of Quality Assurance (QA) evaluation station 116. Based on the information displayed on monitor 117, medical personnel can alter, if required, the calculated 4D adaptive plan so as to be within acceptable parameters. Thus, the QA evaluation station 116 acts as a way to ensure confidence in future real time changes made to the therapy session. In this scenario, the QA evaluation station 116 and the treatment dose tracking and feedback system 500 can be collectively thought of as a 4D planning and control system.

With the above description of the onboard cone-beam computed tomography system 200, megavoltage imaging and radiation therapy system 300, QA evaluation station 116 and the treatment dose tracking and feedback system 500 in mind, the operation of the CBCT IGART system 100 of FIG. 1 can be understood. In particular, the previously described online volumetric imaging information and real time therapy beam parameters are captured from systems 200, 300 and 400 and stored in data storage server 108. The volumetric imaging information and therapy beam parameters are then sent to data monitor job controller 104 that automatically assigns tasks, based on pre-designed treatment schedule and protocol, to each of the work stations 110, 112 and 114 and controls the accomplishment of such tasks. The tasks are stored in temporal job queues 124 for dispatching, based on clinical priorities, to each of the workstations 110, 112 and 114. The clinical priority can be reassigned from a clinical user's request 122 based on the treatment review and evaluation on the physician evaluation/decision making station 106. In addition, the station 106 also provides commands for treatment/plan modification decisions. The modification server 120 receives commands from the station 106 and modifies the ongoing treatment plan, beam or patient position on the system 300 based on the optimized adaptive plan created from the adaptive planning workstation 114.

As shown in FIG. 1, the raw data from server 102 is also sent to a workstation 110. The workstation 110 is dedicated to perform an autosegmentation/registration process on a three-dimensional, volumetric image of a patient generated by cone-beam computed tomography system 200. The raw data from server 102 is also sent to workstation 112 and workstation 114. Workstation 112 performs daily and cumulative treatment dose construction/evaluation from the raw data. Workstation 114 performs adaptive planning from the raw data. These three workstations 110, 112 and 114 perform their tasks automatically in the order of their job queues 126, 128 and 130, respectively.

As shown in FIG. 1, the segmentation/registration, treatment dose construction and adaptive planning information generated from workstations 110, 112 and 114 is sent to the QA evaluation station 116 which interacts with a clinical user to verify and modify, if necessary, the results from the above workstations 110, 112 and 114. The output from QA evaluation station 116 is then stored in derived data server 108.

The QA station 116 provides an update execution status to job execution log server 118 that supplies information whether processing of information is presently occurring, whether processing is completed or whether an error has occurred. Whenever a task of treatment dose construction or adaptive planning modification is completed by workstations 112 and 114, respectively, the evaluation station 116 provides treatment evaluation information which includes both the current treatment status and the completed treatment dose and outcome parameters estimated based on the patient and treatment data from previous treatments. The user at QA evaluation station 116 can then provide commands or a new clinical schedule to the high priority job request server 122 to either request new information or modify clinical treatment schedule. In addition, the user can also make decisions to execute a new adaptive plan or perform a treatment/patient position correction through the server 120.

The CBCT IGART system 100 performs a number of processes, including a kV portal imaging process via kV portal imaging processor/software and an image guided adapted radiation therapy process, among other processes. Further details of the system 100 can be found in U.S. Application No. 12/556,270, filed September 9, 2009.

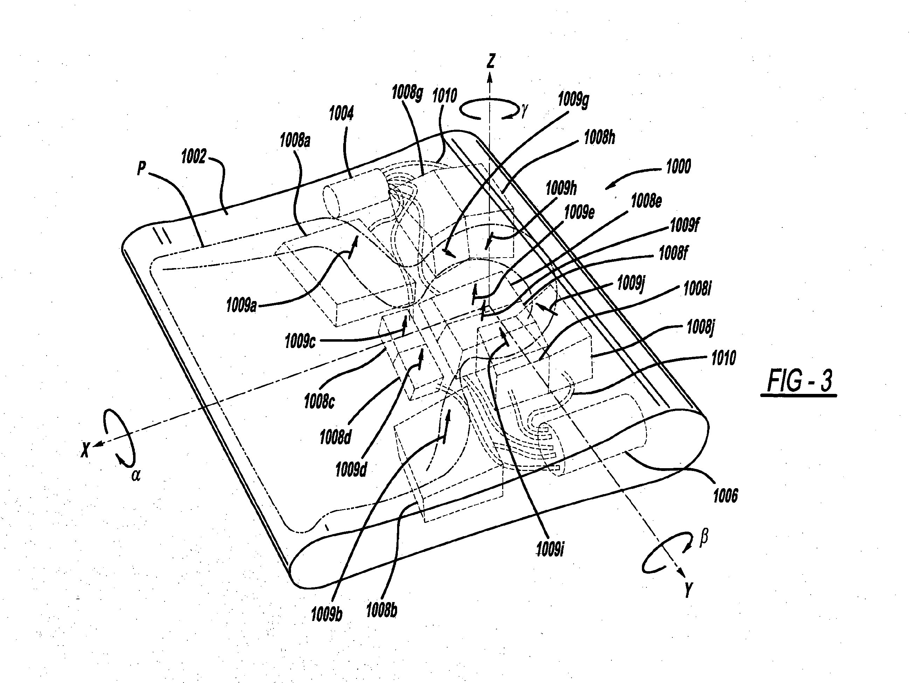

When imaging and treating an anatomical portion of a patient using the CBCT IGART system 100 described above, it is critical that the anatomical portion be immobilized in an appropriate position. Referring now to FIG. 3, one embodiment of the present invention provides a cradle 1000 for positioning an anatomical portion of a patient P during radiation therapy treatment. The cradle 1000 comprises a plurality of expandable fluid chambers 1008, and one or more pressure regulators 1004 and 1006. In one embodiment, shown in FIG. 3, the cradle 1000 further comprises a cover 1002, which houses the fluid chambers 1008a-j and the one or more pressure regulators 1004 and 1006.

Each of the plurality of fluid chambers 1008 is configured to expand to exert a force against one of a plurality of surfaces of the anatomical portion, such that the anatomical portion is held in a fixed position for treatment. Preferably, each of the fluid chambers 1008 exerts a force against a limited area of the anatomical portion. The forces exerted by the fluid chambers 1008 against the surfaces of the anatomical portion are indicated by force vectors 1009. Preferably, the fluid chambers 1008 are arranged such that at least two of the force vectors 1009 substantially oppose one another, such that the anatomical portion is immobilized in the in the cradle 1000.

The various components of the cradle 1000, namely the fluid chambers 1008 and the cover 1002 (if applicable), are typically made of materials that do not attenuate x-ray or particle radiation. The fluid chambers 1008 may be constructed from any suitable flexible, elastic, and fluid-tight material or membrane that will allow them to expand when fluid is supplied and contract when fluid is withdrawn. The fluid chambers 1008 may be constructed to expand and contract in all directions when fluid is supplied and withdrawn, or they may have an accordion-like construction that allows them to expand and contract primarily along a single axis (not shown) when fluid is supplied or withdrawn. Preferably, the fluid chambers 1008 each expand and contract along a single axis. More preferably, each of the fluid chambers 1008 expands along a single axis parallel to its respective force vector 1009.

The pressure regulators 1004 and 1006 are configured to adjust the pressure of each of the fluid chambers 1008 independently of every other fluid chamber 1008 by supplying fluid to or withdrawing fluid from each of the fluid chambers. The pressure regulators 1004 and 1006 may each include a pump (not shown) for supplying fluid to the fluid chambers 1008 and a vent (not shown) for withdrawing fluid from the fluid chambers 1008. However, other suitable pressure regulators known to those having ordinary skill in the relevant art may also be employed without falling outside the scope of the present invention. The pressure regulators 1004 and 1006 may be connected to the fluid chambers 1008 by tubing sections 1010. However, other suitable arrangements and means of attachment may also be employed. Finally, while the embodiment shown in FIG. 3 includes two pressure regulators 1004 and 1006, any other suitable number of pressure regulators may be employed. For example, a single pressure regulator may be employed. Alternatively, a separate pressure regulator may be employed for each of the fluid chambers 1008.

Because the pressure regulators 1004 and 1006 are configured to adjust the pressure of each of the fluid chambers 1008 independently of every other fluid chamber 1008, the cradle 1000 enables the operator to adjust the position of the anatomical portion of the patient P in up to all six degrees of freedom. Specifically, by supplying an appropriate amount of fluid to, or withdrawing an appropriate amount of fluid from, each of the fluid chambers 1008, the anatomical portion can be translated along a longitudinal axis x, a lateral axis y, or a vertical axis z. Moreover, the anatomical portion can be rotated about the longitudinal axis x (as indicated by the arrow α), about the lateral axis y (as indicated by the arrow β), or about the vertical axis z (as indicated by the arrow y).

As will be understood by one having ordinary skill in the relevant art, the cradle 1000 may be configured to position any suitable anatomical portion of a patient, by selecting a suitable arrangement of the fluid chambers 1008, without falling outside the scope of the present invention. For example, the cradle 1000 may be configured to position a patient's head, arm, hand, pelvis, leg, or foot. In the embodiment shown in FIG. 3, the cradle is configured to position a patient's head. Moreover, the cradle 1000 may be configured to position the anatomical portion of the patient when the patient is in a standing, seated, prone, or supine position. In the embodiment shown in FIG. 3, the cradle 1000 is configured to position the patient's head when the patient is in a supine position on a couch during treatment.

In the embodiment shown in FIG. 3, the cradle 1000 includes ten fluid chambers 1008a-j. Specifically, this embodiment of the cradle 1000 includes two first fluid chambers 1008c and 1008d configured to exert a first force against the back of the patient's neck, two second fluid chambers 1008e and 1008f configured to exert a second force against the back of the patient's head, two third fluid chambers 1008i and 1008j configured to exert a third force against the left side of the patient's head, two fourth fluid chambers 1008g and 1008h configured to exert a fourth force against the right side of the patient's head, and two fifth fluid chambers 1008a and 1008b configured to exert a fifth force against the backs of the patient's shoulders.

The first fluid chambers 1008c and 1008d are configured to exert an increased first force against the back of the patient's neck when fluid is supplied to the first fluid chambers 1008c and 1008d and to exert a decreased first force against the back of the patient's neck when fluid is withdrawn from the first fluid chambers 1008c and 1008d. The first fluid chambers 1008c and 1008d may include a right neck fluid chamber 1008c and a left neck fluid chamber 1008d. The right neck fluid chamber 1008c may be configured to exert an increased force against the back right side of the patient's neck, as indicated by force vector 1009c, when fluid is supplied to the right neck fluid chamber 1008c and to exert a decreased force against the back right side of the patient's neck when fluid is withdrawn from the right neck fluid chamber 1008c. The left neck fluid chamber 1008d may be configured to exert an increased force against the back left side of the patient's neck, as indicated by force vector 1009d, when fluid is supplied to the left neck fluid chamber 1008d and to exert a decreased force against the back left side of the patient's neck when fluid is withdrawn from the left neck fluid chamber 1008d.

The second fluid chambers 1008e and 1008f are configured to exert an increased second force against the back of the patient's head when fluid is supplied to the second fluid chambers 1008e and 1008f and to exert a decreased second force against the back of the patient's head when fluid is withdrawn from the second fluid chambers 1008e and 1008f. The second fluid chambers 1008e and 1008f may include a right head fluid chamber 1008e and a left head fluid chamber 1008f. The right head fluid chamber 1008e may be configured to exert an increased force against the back right side of the patient's head, as indicated by force vector 1009e, when fluid is supplied to the right head fluid chamber 1008e and to exert a decreased force against the back right side of the patient's head when fluid is withdrawn from the right head fluid chamber 1008e. The left head fluid chamber 1008f may be configured to exert an increased force against the back left side of the patient's head, as indicated by force vector 1009f, when fluid is supplied to the left head fluid chamber 1008f and to exert a decreased force against the back left side of the patient's head when fluid is withdrawn from the left head fluid chamber 1008f.

The third fluid chambers 1008i and 1008j are configured to exert an increased third force against the left side of the patient's head when fluid is supplied to the third fluid chambers 1008i and 1008j and to exert a decreased third force against the left side of the patient's head when fluid is withdrawn from the third fluid chambers 1008i and 1008j. The third fluid chambers 1008i and 1008j may include a left temple fluid chamber 1008i and a top left head fluid chamber 1008j. The left temple fluid chamber 1008i may be configured to exert an increased force against the left temple of the patient's head, as indicated by force vector 1009i, when fluid is supplied to the left temple fluid chamber 1008i and to exert a decreased force against the left temple of the patient's head when fluid is withdrawn from the left temple fluid chamber 1008i. The top left head fluid chamber 1008j may be configured to exert an increased force against the left side of the patient's head above the patient's left temple, as indicated by force vector 1009j, when fluid is supplied to the top left head fluid chamber 1008j and to exert a decreased force against the left side of the patient's head above the patient's left temple when fluid is withdrawn from the top left head fluid chamber 1008j.

The fourth fluid chambers 1008g and 1008h are configured to exert an increased fourth force against the right side of the patient's head when fluid is supplied to the fourth fluid chambers 1008g and 1008h and to exert a decreased fourth force against the right side of the patient's head when fluid is withdrawn from the fourth fluid chambers 1008g and 1008h. The fourth fluid chambers 1008g and 1008h may include a right temple fluid chamber 1008g and a top right head fluid chamber 1008h. The right temple fluid chamber 1008g may be configured to exert an increased force against the right temple of the patient's head, as indicated by force vector 1009g, when fluid is supplied to the right temple fluid chamber 1008g and to exert a decreased force against the right temple of the patient's head when fluid is withdrawn from the right temple fluid chamber 1008g. The top right head fluid chamber 1008h may be configured to exert an increased force against the right side of the patient's head above the patient's right temple, as indicated by force vector 1009h, when fluid is supplied to the top right head fluid chamber 1008h and to exert a decreased force against the right side of the patient's head above the patient's right temple when fluid is withdrawn from the top right head fluid chamber 1008h.

The fifth fluid chambers 1008a and 1008b are configured to exert an increased fifth force against the backs of the patient's shoulders when fluid is supplied to the fifth fluid chambers and to exert a decreased fifth force against the backs of the patient's shoulders when fluid is withdrawn from the fifth fluid chambers. The fifth fluid chambers 1008a and 1008b may include a right shoulder fluid chamber 1008a and a left shoulder fluid chamber 1008b. The right shoulder fluid chamber 1008a may be configured to exert an increased force against the back of the patient's right shoulder, as indicated by force vector 1009a, when fluid is supplied to the right shoulder fluid chamber 1008a and to exert a decreased force against the back of the patient's right shoulder when fluid is withdrawn from the right shoulder fluid chamber 1008a. The left shoulder fluid chamber 1008b may be configured to exert an increased force against the back of the patient's left shoulder, as indicated by force vector 1009b, when fluid is supplied to the left shoulder fluid chamber 1008b and to exert a decreased force against the back of the patient's left shoulder when fluid is withdrawn from the left shoulder fluid chamber 1008b.

Although the embodiment of the cradle 1000 shown in FIG. 3 has ten fluid chambers 1008a-j, those having ordinary skill in the relevant art will understand that the cradle may have any suitable number of fluid chambers 1008 without falling outside the scope of the present invention. Moreover, the fluid chambers 1008 may be arranged in any suitable configuration without falling outside the scope of the present invention.

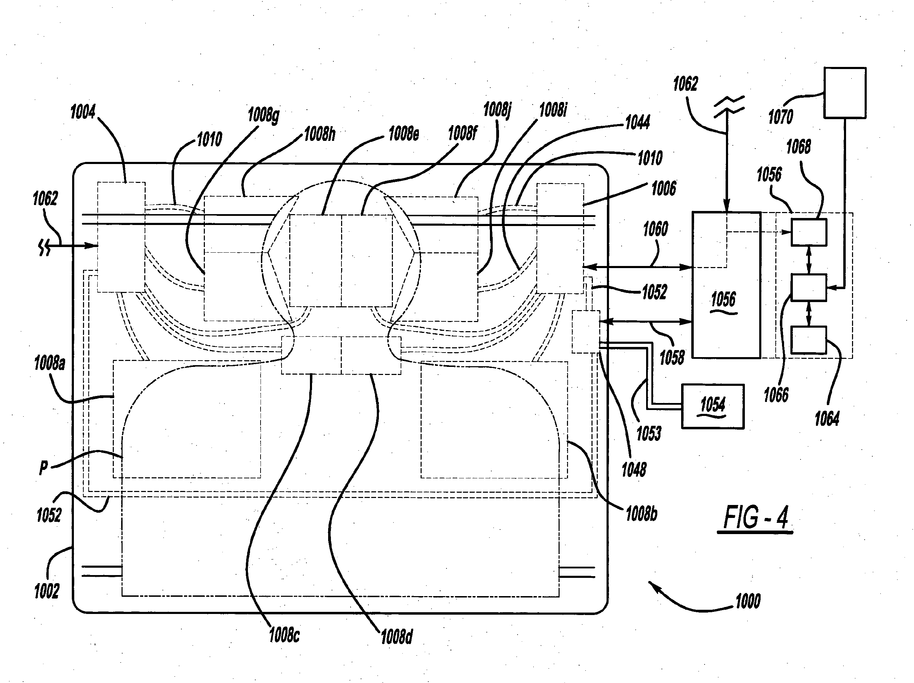

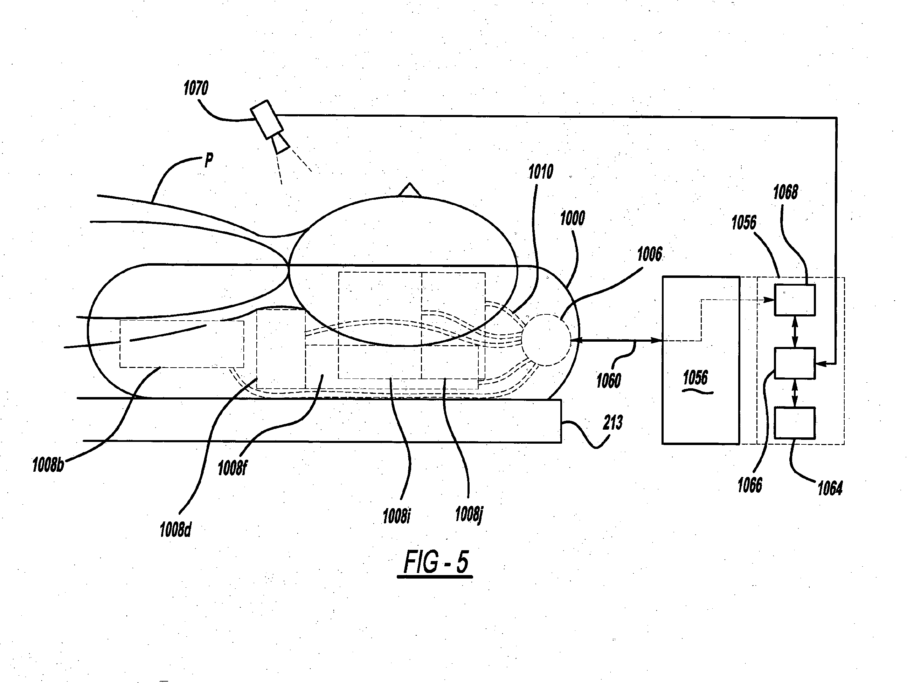

Referring now to FIGs. 4 and 5, the present invention also provides a system for positioning an anatomical portion of a patient during radiation therapy treatment. The system comprises the cradle 1000, described above with reference to FIG. 3, and a control unit 1056. The control unit is configured to instruct the pressure regulators 1004 and 1006 to adjust the pressure of each of the fluid chambers 1008 to a desired pressure, such that the anatomical portion of the patient is held in a desired position. The system may further comprise a fluid source 1054 configured to supply fluid through a conduit 1053 to a valve 1048, where the valve 1048 is connected to the pressure regulators 1004 and 1006 by the conduits 1052.

The system may be configured to hold the anatomical portion in any desired position. In some embodiments, the desired position is a predetermined position. For example, the desired position in a second treatment session may be substantially similar to a first position of the anatomical portion during a first session. As used herein, the term "first session" may refer to any treatment session, regardless of how many treatment sessions have preceded or will follow such treatment session, and may also refer to a pretreatment planning session. As used herein, the term "second treatment session" may refer to any treatment session following a first session.

Like the stand-alone cradle 1000 described above, the system may be configured to position any suitable anatomical portion of a patient, by selecting a suitable arrangement of the fluid chambers 1008 of the cradle 1000, without falling outside the scope or spirit of the present invention. For example, the system may be configured to position a patient's head, arm, hand, pelvis, leg, or foot. In the embodiment shown in FIGs. 4 and 5, the cradle is configured to position a patient's head when the patient is in a supine position on a couch during radiation therapy treatment.

The control unit 1056 may communicate with the pressure regulators 1004 and 1006 and optionally with the valve 1048 by any means known to those having ordinary skill in the relevant art. For example, the control unit 1056 may communicate with the pressure regulators 1004 and 1006 and optionally with the valve 1048 by means of wires 1062, 1060, and 1058, respectively. Alternatively, the control unit may communicate wirelessly with the pressure regulators 1004 and 1006 and optionally with the valve 1048.

In some embodiments, the control unit 1056 may be configured to receive manual inputs from an operator to adjust the pressure of one or more of the fluid chambers 1008, and to relay these instructions to the pressure regulators 1004 and 1006. In other embodiments, which will be referred to as the "automated system," the control unit 1056 may be configured determine the desired pressure of each of the fluid chambers 1008 in an automated fashion. The control unit 1056 of the automated system may be configured to also receive manual inputs.

In the automated system, the control unit includes a memory means 1064, a microprocessor 1066, and a signaling means 1068. The memory means 1064 may be any device known by those having ordinary skill in the relevant art to be useful for data storage. Thus, the memory means may be a computer server, a computer hard disk, a computer floppy disk, a CD-ROM, a flash drive, or any other suitable means of data storage. The microprocessor 1066 may be any computer processor known by those having ordinary skill in the relevant art to be useful for data manipulation. The microprocessor 1066 may communicate with the memory means 1064 by any means known to those having ordinary skill in the relevant art, including by wired or wireless communication. The signaling means 1068 may be any device known by those having ordinary skill in the relevant art to be useful for sending instructions from the microprocessor 1066 to the pressure controllers 1004 and 1006 and optionally the valve 1048. As discussed above, the signaling means 1068 of the control unit 1056 may communicate with the pressure regulators 1004 and 1006 and optionally with the valve 1048 through wires 1062, 1060, and 1058, respectively, or by wireless communication.

In a first embodiment of the automated system, the system is configured to position the anatomical portion in a predetermined position by adjusting the pressure of each of the fluid chambers to a predetermined pressure. In a first session, the predetermined pressure of each of the fluid chambers may be determined by computer modeling of the pressure needed to be employed to place the patient's anatomical portion in the predetermined position. In a second treatment session, the predetermined position may be determined by reference to a first position of the anatomical portion during a first session. In this case, the predetermined pressure of each of the fluid chambers during the second treatment session may be substantially similar to the pressure of each of the fluid chambers during the first session.

In the first embodiment of the automated system, the memory means 1064 is configured to store predetermined inflation data for each of the fluid chambers 1008. The predetermined inflation data for each of the fluid chambers 1008 represents a predetermined pressure of each of the fluid chambers 1008 corresponding to the predetermined position of the anatomical portion. In a second treatment session, the predetermined pressure of each of the fluid chambers 1008 may be substantially similar to the pressure of each of the fluid chambers 1008 during a first session.

In the first embodiment of the automated system, the microprocessor 1066 is configured to receive the predetermined inflation data for each fluid chamber from the memory means 1064 and to determine the predetermined pressure of each of the fluid chambers 1008 based on the predetermined inflation data for that fluid chamber.

In the first embodiment of the automated system, the signaling means 1068 is configured to receive instructions regarding the predetermined pressure of each of the fluid chambers 1008 from the microprocessor 1066 and to send an inflation signal to the pressure regulators 1004 and 1006 and optionally to the valve 1048. The inflation signal instructs the pressure regulators 1004 and 1006 to adjust the pressure of each of the fluid chambers 1008 to the predetermined pressure.

In a second embodiment of the automated system, most clearly shown in FIG. 5, the system is configured to position the anatomical portion in a predetermined position using real-time patient surface measurement or other onboard imaging. The system positions the anatomical portion in the predetermined position by comparing the current position of the anatomical portion, represented by a current image, which may be an in-room optical, x-ray, or MRI image, with the predetermined position of the anatomical portion, represented by a first image of the anatomical portion. The first image may have been acquired during a first session.

In the second embodiment of the automated system, the system includes an imaging device 1070. The imaging device 1070 may be any device known by those having ordinary skill in the relevant art to be suitable for acquiring an image of an anatomical portion of a patient. For example, the imaging device 1070 may be a video camera, a computed tomography scanner, or an MRI imager. Preferably, the imaging device 1070 is a video camera. More preferably, the imaging device 1070 is a video camera suitable for acquiring a moving image. The imaging device 1070 is configured to acquire a current image, which represents the current position of the anatomical portion. Preferably, the current image is a moving image of the current position of the anatomical portion.

In the second embodiment of the automated system, the memory means 1064 is configured to store a first image, which represents the predetermined position of the anatomical portion. As noted above, the first image may have been acquired during a first session, such that the predetermined position of the anatomical portion shown in the first image is a first position of the anatomical portion during the first session. The first image may have been acquired by any means known by those having ordinary skill in the relevant art, including but not limited to video imaging or computed tomography imaging. Preferably, the first image is a still image of the anatomical portion.

In the second embodiment of the automated system, the microprocessor 1066 is configured to receive the first image from the memory means 1064 and the current image from the imaging device 1070. The microprocessor 1066 is further configured, by comparing the current position of the anatomical portion (as represented by the current image) to the predetermined position of the anatomical portion (as represented by the first image), to determine an effective pressure of each of the fluid chambers 1008 to adjust the current position of the anatomical portion, such that the current position of the anatomical portion is substantially similar to the predetermined position of the anatomical portion.

In comparing the current position of the anatomical portion to the predetermined position of the anatomical portion, the microprocessor 1066 preferably compares the current and predetermined positions of certain anatomical landmarks of the anatomical portion. As used herein, the term "anatomical landmark" refers to a feature of an anatomical portion whose position will not change as a result of foreseeable changes in the overall shape of the anatomical portion, e.g. changes due to patient weight gain or weight loss, changes due to patient hydration levels, or changes due to changes in patient attire or hair style. For example, where the anatomical portion is the patient's head, suitable anatomical landmarks may include the tip of the patient's nose and the frontal bone of the patient's cranium.

In the second embodiment of the automated system, the signaling means 1068 is configured to receive instructions from the microprocessor 1066 regarding the effective pressure of each of the fluid chambers 1008. The signaling means 1068 is also configured to send an adjustment signal to the pressure regulators 1004 and 1006 and optionally to the valve 1048. The adjustment signal instructs the pressure regulators 1004 and 1006 to adjust the pressure of each of the fluid chambers 1008 to the effective pressure.

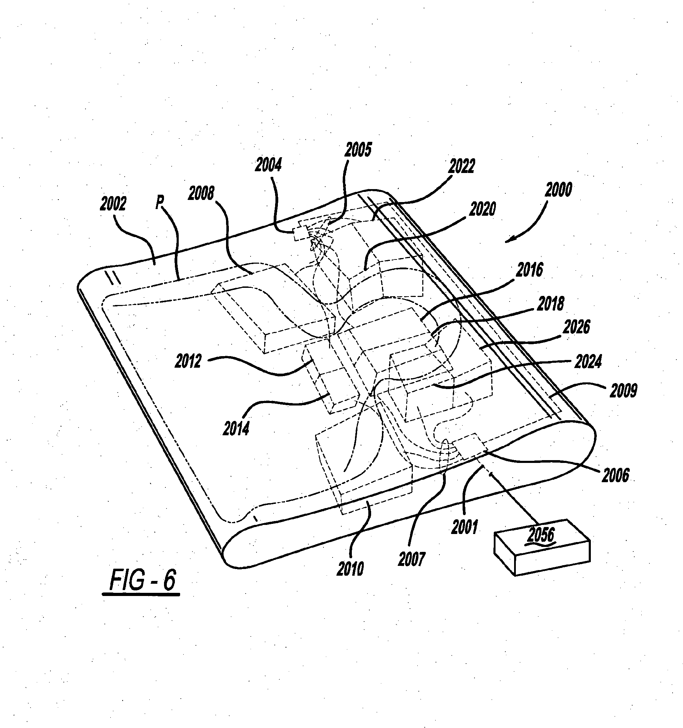

Turning now to FIG. 6, another cradle 2000 embodying the principles of the present invention is illustrated. As its primary components the cradle 2000 includes a flexible cover 2002 and a set of 10 fluid chambers 2008, 2010, 2012, 2014, 2016, 2018, 2020, 2022, 2024, and 2026, each of which is made of a suitable flexible material or membrane.

The fluid chambers 2008 and 2010 primarily provide support to the patient's shoulder regions; the fluid chambers 2012 and 2014 provide support mostly to the patient's neck region; the fluid chambers 2016 and 2018 provide support to the back of the patient's head; and the fluid chambers 2020, 2022, 2024, and 2026 provide lateral support to either side of the patient's head.

Also enclosed in the cover 2002 is a pair of microprocessors 2004 and 2006. The microprocessor 2006 is connected to a control unit 2056 with an electrical connection 2001 and to the other microprocessor 2004 with an electrical connection 2009. In some implementations the microprocessor 2004 is connected directly to the control unit 2056.

Each of the fluid chambers 2008, 2010, 2012, 2014, 2016, 2018, 2020, 2022, 2024, and 2026 operates as an individual electrically controlled pressure regulator or pump. Accordingly, the fluid chambers communicate with the microprocessors 2004 and 2006 through a set of electrical connections 2005 and a set of electrical connections 2007, respectively. In particular, each of the fluid chambers 2008, 2012, 2016, 2020, and 2022 receives individual signals from the microprocessor 2004 through a respective electrical connection of the set of electrical connections 2005, and each of the fluid chambers 2010, 2014, 2018, 2024, and 2026 receives individual signals from the microprocessor 2006 through a respective electrical connection of the set of electrical connections 2007. The signals instruct each respective air chamber to achieve a desired pressure.

The microprocessors 2004 and 2006 receive signals from and provide feedback to the control unit 2056. In certain implementations, the communication between the control unit 2056 and the microprocessors 2004 and 2006 is wireless. In yet other implementations, the control unit 2056 communicates directly with the fluid chambers 2008, 2010, 2012, 2014, 2016, 2018, 2020, 2022, 2024, and 2026 through a set of electrical connections or through a wireless system. The control unit 2056 may receive instructions directly from an operator and/or the control unit 2056 may comprise an automated system analogous to the control unit 1056 described above.

When the cradle 2000 is in use, the fluid chambers 2008, 2010, 2012, 2014, 2016, 2018, 2020, 2022, 2024, and 2026 cradle the patient's head as each of the air chambers inflates individual. The control unit 2056 sends instructions to the microprocessors 2004 and 2006 to adjust the pressures in the various fluid chambers 2008, 2010, 2012, 2014, 2016, 2018, 2020, 2022, 2024, and 2026 to provide immobilization and/or positioning of the patient's head. The cradle 2000 can be employed to immobilize other body parts or anatomical portions, as well, during treatment of the patient. The cradle 2000 also allows for movement of the patient during treatment, for example, when the patient changes position.

Although the cradle 2000 is shown with ten fluid chambers, other implementations of the cradle 2000 may use fewer chambers or more than ten chambers. The registration of the patient can take place through the use of a video camera which therefore does not involve any dose exposure. The various components of the cradle 2000 are typically made of materials that do not attenuate radiation. Software can be implemented in the control unit 2056 to adjust pressure in the various fluid chambers as needed to position and reposition the patient.

In proton therapy systems, a fixed beam is typically formed with a large accelerator, and therefore the beam is difficult to reposition between therapy sessions or during a particular therapy session. Further, certain anatomical features of a patient are difficult to treat using a proton beam on a conventional table or couch. Various implementations of the cradles 1000 and 2000 are suitable for use in such proton therapy systems and, further, can be adapted for a sitting patient on a chair-type system.

The present disclosure provides a method of positioning an anatomical portion of a patient during treatment. The method generally comprises the steps of (1) placing the anatomical portion of the patient in a cradle, such as the cradle described above with reference to FIG. 3, having a plurality of expandable fluid chambers; and (2) adjusting the pressure of each of the fluid chambers to a desired pressure by supplying fluid to or withdrawing fluid from each of the fluid chambers, such that the anatomical portion is held in a desired position. The expandable fluid chambers of the cradle employed in the method, like those of the cradle described above with reference to FIG. 3, are configured to exert a force against one of a plurality of surfaces of the anatomical portion. At least two of the plurality of fluid chambers may exert substantially opposing forces against the anatomical portion, such that the anatomical portion is immobilized in the cradle.

As will be understood by those having ordinary skill in the relevant art, the present method may be employed to position various anatomical portions of a patient, including the patient's head, arm, hand, pelvis, leg, or foot. Preferably, the method is employed to position the patient's head. However, it will be understood that the method may be employed to position any suitable anatomical portion of a patient.

It will be further understood that any suitable fluid may be employed in the method for the purpose of inflating the fluid chambers. Preferably, the fluid is a radiotransparent fluid, such as air, nitrogen, argon, or any other suitable radiotransparent fluid. More preferably, the fluid is air, nitrogen, or argon. Most preferably, the fluid is air. However, it will be understood that any fluid may be employed.

The method described above may be employed to position an anatomical portion of a patient during either a first session or a second treatment session. As noted above, the term "first session" may refer to any treatment session, regardless of how many treatment sessions have preceded or will follow such treatment session, and may also refer to a pretreatment planning session. As used herein, the term "second treatment session" may refer to any treatment session following a first session.

In a first embodiment the method is used to position an anatomical portion of a patient during a first session. In this embodiment, the pressure of each of the fluid chambers may be controlled manually by a medical practitioner or technician. The pressure of each of the fluid chambers may be adjusted to a desired pressure by supplying fluid to or withdrawing fluid from each of the fluid chambers individually until a desired position of the anatomical portion is achieved. In this embodiment, the desired position of the anatomical portion may be any position in which the patient is comfortable and which is suitable for the administration of the relevant treatment.

In a second embodiment the method is used to position an anatomical portion of a patient during a second treatment session. The desired position of the anatomical portion during the second treatment session may be substantially similar to a first position of the anatomical portion during a preceding first session.

In a first variation of the second embodiment of the method, the desired position may be achieved during the second treatment session by adjusting the pressure of each of the fluid chambers to be substantially similar to the pressure of that fluid chamber during the first session.

In a second variation of the second embodiment of the method, the desired position may be achieved by comparing the current position of the anatomical portion during the second treatment session to the first position of the anatomical portion during the first session, as embodied in a first image of the anatomical portion acquired during the first session. The pressure of each of the fluid chambers is then adjusted to an effective pressure by supplying fluid to or withdrawing fluid from the fluid chambers to adjust the current position of the anatomical portion, such that the current position of the anatomical portion matches the first position of the anatomical portion shown in the first image. This variation of the second embodiment of the method, therefore, comprises the following steps:
1) Acquiring a first image of the anatomical portion during a first session, the first image representing the first position of the anatomical portion;
2) At the outset of the second treatment session, placing the anatomical portion of the patient in a cradle, such as the cradle described above with reference to FIG. 3, having a plurality of expandable fluid chambers;
3) Comparing the current position of the anatomical portion to the first position of the anatomical portion; and
4) Adjusting the pressure of each of the fluid chambers to an effective pressure to adjust the current position of the anatomical portion, such that the current position of the anatomical portion is substantially similar to the first position of the anatomical portion.

The step of comparing the current position of the anatomical portion to the first position of the anatomical portion, and the step of adjusting the pressure of each of the fluid chambers to an effective pressure, may be performed manually or by an automated system, such as the system described above with reference to FIGs. 4 and 5.

In performing the steps manually, an operator may visually compare the current position of the anatomical portion to the first position of the anatomical portion embodied in the first image. The operator may then manually adjust the pressure of each of the fluid chambers by causing fluid to be supplied to or withdrawn from each of the fluid chambers to adjust the current position of the anatomical portion, such that the current position of the anatomical portion is substantially similar to the first position of the anatomical portion.

To perform the steps using an automated system, the operator may employ the system described above with reference to FIGs. 4 and 5. The imaging device of the automated system may acquire a current image representing the current position of the anatomical portion. The microprocessor of the automated system may then compare the current position of the anatomical portion (embodied in the current image) to the first position of the anatomical portion (embodied in the first image). The signaling means of the automated system may then instruct the pressure regulators to adjust the current position of the anatomical portion, such that the current position of the anatomical portion is substantially similar to the first position of the anatomical portion.

While the present invention has been described in terms of certain preferred embodiments, it will be understood that the invention is not limited to the disclosed embodiments, as those having skill in the art may make various modifications without departing from the scope of the following claims.

## Claims

1. A cradle (1000; 2000) for positioning an anatomical portion of a patient during radiation therapy treatment, the cradle (1000;2000) comprising:
a plurality of expandable fluid chambers (1008a-j; 2010; 2012; 2014; 2016; 2018; 2020; 2022; 2024; 2026), each of the plurality of fluid chambers (1008a-j; 2010; 2012; 2014; 2016; 2018; 2020; 2022; 2024; 2026) being configured to exert a force against one of a plurality of surfaces of the anatomical portion; and
a pressure regulator (1004; 1006; 2010; 2012; 2014; 2016; 2018; 2020; 2022; 2024; 2026) configured to adjust the pressure of each of the fluid chambers (1008a-j; 2010; 2012; 2014; 2016; 2018; 2020; 2022; 2024; 2026) independently of every other fluid chamber by supplying fluid to or withdrawing fluid from each of the fluid chambers (1008a-j; 2010; 2012; 2014; 2016; 2018; 2020; 2022; 2024; 2026); **characterised in that**
at least two of the plurality of fluid chambers (1008a-j; 2010; 2012; 2014; 2016; 2018; 2020; 2022; 2024; 2026) are configured to exert substantially opposing forces against the anatomical portion, such that the anatomical portion is immobilized in the cradle (1000; 2000).

2. The cradle (1000; 2000) of claim 1, wherein the cradle (1000; 2000) is positioned for a patient in a supine position on a couch during treatment.

3. The cradle (1000) of claim 1, wherein the plurality of expandable fluid chambers (1008a-j) comprises:
a first fluid chamber (1008c; 1008d) configured to exert a first force against the back of the patient's neck;
a second fluid chamber configured to exert a second force against the back of the patient's head (1008e; 1008f);
a third fluid chamber (1008i; 1008j) configured to exert a third force against the left side of the patient's head; and
a fourth fluid chamber (1008g; 1008h) configured to exert a fourth force against the right side of the patient's head.

4. The cradle (1000) of claim 3, wherein the first fluid chamber (1008c; 1008d) is configured such that the first force increases in magnitude when fluid is supplied to the first fluid chamber (1008c; 1008d) and decreases in magnitude when fluid is withdrawn from the first fluid chamber (1008c; 1008d).

5. A system for positioning an anatomical portion of a patient during radiation therapy treatment, the system comprising:
the cradle (1000; 2000) according to one of the preceding claims, and
a control unit (1056; 2056) configured to instruct the pressure regulator (1004; 1006; 2010; 2012; 2014; 2016; 2018; 2020; 2022; 2024; 2026) to adjust the pressure of each of the fluid chambers (1008a-j; 2010; 2012; 2014; 2016; 2018; 2020; 2022; 2024; 2026), such that the anatomical portion is held in a desired position.

6. The system of claim 5, wherein the desired position is a predetermined position.

7. The system of claim 6, wherein the control unit (1056) further comprises:
a memory means (1064), the memory means (1064) being configured to store predetermined inflation data for each of the fluid chambers (1008a-j), the predetermined inflation data representing a predetermined pressure of each of the fluid chambers (1008a-j), the predetermined pressures corresponding to the predetermined position of the anatomical portion;
a microprocessor (1066), the microprocessor (1066) being configured to receive the predetermined inflation data from the memory means (1064) and based on the predetermined inflation data determine the predetermined pressure of each of the fluid chambers (1008a-j); and
a signaling means (1068) configured to receive instructions from the microprocessor (1066) regarding the predetermined pressure of each of the fluid chambers (1008a-j) and send an inflation signal to the pressure regulator (1004; 1006), the inflation signal instructing the pressure regulator (1004; 1006) to adjust the pressure of each of the fluid chambers (1008a-j) to the predetermined pressure.

8. The system of claim 6, wherein the predetermined position of the anatomical portion during a second treatment session is substantially similar to a first position of the anatomical portion during a first session.

9. The system of claims 5 and 8, further comprising:
an imaging device (1070), the imaging device (1070) being configured to acquire a current image, the current image representing a current position of the anatomical portion;
wherein the control unit (1056) further comprises:
a memory means (1064), the memory means (1064) being configured to store a first image, the first image having been acquired during a first session, the first image representing a first position of the anatomical portion during the first session;
a microprocessor (1066), the microprocessor (1066) being configured to receive the first image from the memory means (1064) and the current image from the imaging device (1070) and based on the first image and the current image determine an effective pressure of each of the fluid chambers (1008a-j) to adjust the current position of the anatomical portion, such that the current position of the anatomical portion is substantially similar to the first position of the anatomical portion; and
a signaling means (1068), the signaling means (1068) being configured to receive instruction from the microprocessor (1066) regarding the effective pressure of each of the fluid chambers (1008a-j) and send an adjustment signal to the pressure regulator (1004;1006), the adjustment signal instructing the pressure regulator (1004;1006) to adjust the pressure of each of the fluid chambers (1008a-j) to the effective pressure.

10. The system of claim 9, wherein the imaging device (1070) is a video camera, and the current image is a moving image.

## Patentansprüche

1. Halter (1000; 2000) zur Positionierung eines anatomischen Teils eines Patienten während einer strahlentherapeutischen Behandlung, wobei der Halter (1000; 2000) Folgendes umfasst:
eine Vielzahl von expandierbaren Fluidkammern (1008a-j; 2010; 2012; 2014; 2016; 2018; 2020; 2022; 2024; 2026), wobei jede der Vielzahl von expandierbaren Fluidkammern (1008a-j; 2010; 2012; 2014; 2016; 2018; 2020; 2022; 2024; 2026) zum Ausüben einer Kraft gegen eine einer Vielzahl von Flächen des anatomischen Teils ausgelegt ist; und
einen Druckregler (1004; 1006; 2010; 2012; 2014; 2016; 2018; 2020; 2022; 2024; 2026), der zur Anpassung des Drucks jeder der Fluidkammern (1008a-j; 2010; 2012; 2014; 2016; 2018; 2020; 2022; 2024; 2026) unabhängig von jeder jeweils anderen Fluidkammer ausgelegt ist, indem er jeder der Fluidkammern (1008a-j; 2010; 2012; 2014; 2016; 2018; 2020; 2022; 2024; 2026) Fluid zuführt oder Fluid davon abzieht;
**dadurch gekennzeichnet, dass** zumindest zwei der Fluidkammern (1008a-j; 2010; 2012; 2014; 2016; 2018; 2020; 2022; 2024; 2026) ausgelegt sind, im Wesentlichen entgegengesetzte Kräfte gegen den anatomischen Teil auszuüben, so dass der anatomische Teil in dem Halter (1000; 2000) immobilisiert wird.

2. Halter (1000; 2000) nach Anspruch 1, worin der Halter (1000; 2000) für einen Patienten in Rückenlage auf einer Couch während einer Behandlung positioniert ist.

3. Halter (1000) nach Anspruch 1, worin die Vielzahl von expandierbaren Fluidkammern (1008a-j) Folgendes umfasst:
eine erste Fluidkammer (1008c; 1008d), die zum Ausüben einer Kraft gegen die Rückseite des Halses des Patienten ausgelegt ist;
eine zweite Fluidkammer (1008e, 1008f), die zum Ausüben einer zweiten Kraft gegen die Rückseite des Kopfes des Patienten ausgelegt ist;
eine dritte Fluidkammer (1008i, 1008j), die zum Ausüben einer dritten Kraft gegen die linke Kopfseite des Patienten ausgelegt ist; und
eine vierte Fluidkammer (1008g; 1008h), die zum Ausüben einer vierten Kraft gegen die rechte Kopfseite des Patienten ausgelegt ist.

4. Halter (1000) nach Anspruch 3, worin die erste Fluidkammer (1008c; 1008d) so ausgelegt ist, dass die Größe der ersten Kraft zunimmt, wenn der ersten Fluidkammer (1008c; 1008d) Fluid zugeführt wird, und abnimmt, wenn der ersten Fluidkammer (1008c; 1008d) Fluid entzogen wird.

5. System zur Positionierung eines anatomischen Teils eines Patienten während einer strahlentherapeutischen Behandlung, wobei das System Folgendes umfasst:
den Halter (1000; 2000) nach einem der vorhergehenden Ansprüche,
und
eine Steuereinheit (1056; 2056), die ausgelegt ist den Druckregler (1004; 1006; 2010; 2012; 2014; 2016; 2018; 2020; 2022; 2024; 2026) anzuweisen, den Druck in jeder der Fluidkammern (1008a-j; 2010; 2012; 2014; 2016; 2018; 2020; 2022; 2024; 2026) anzupassen, so dass der anatomische Teil in einer gewünschten Stellung gehalten wird.

6. System nach Anspruch 5, worin die gewünschte Stellung eine vorbestimmte Stellung ist.

7. System nach Anspruch 6, worin die Steuereinheit (1056) ferner Folgendes umfasst:
ein Speichermittel (1064), wobei das Speichermittel (1064) zum Speichern vorbestimmter Aufblasdaten für jede der Fluidkammern (1008a-j) ausgelegt ist, wobei die vorbestimmten Aufblasdaten einen vorbestimmten Druck jeder der Fluidkammern (1008a-j) darstellen, wobei die vorbestimmten Drücke der vorbestimmten Stellung des anatomischen Teils entsprechen;
einen Mikroprozessor (1066), wobei der Mikroprozessor (1066) zum Empfangen der vorbestimmten Aufblasdaten von dem Speichermittel (1064) und zum Bestimmen, auf Basis der vorbestimmten Aufblasdaten, des vorbestimmten Drucks jeder der Fluidkammern (1008a-j) ausgelegt ist; und
ein Signalisierungsmittel (1068), das zum Empfangen von Anweisungen von dem Mikroprozessor (1066) bezüglich des vorbestimmten Drucks jeder der Fluidkammern (1008a-j) und zum Senden eine Aufblassignals an den Druckregler (1004; 1006) ausgelegt ist, wobei das Signal den Druckregler (1004; 1006) anweist, den Druck jeder der Fluidkammern (1008a-j) an den vorbestimmten Druck anzupassen.

8. System nach Anspruch 6, worin die vorbestimmte Stellung des anatomischen Teils während einer zweiten Behandlungssitzung im Wesentlichen einer ersten Stellung des anatomischen Teils während einer ersten Sitzung ähnelt.

9. System nach den Ansprüchen 5 und 8, ferner umfassend:
eine Bildgebungsvorrichtung (1070), wobei die Bildgebungsvorrichtung (1070) zur Erfassung eines aktuellen Bildes ausgelegt ist, wobei das aktuelle Bild eine aktuelle Stellung des anatomischen Teils darstellt;
worin die Steuereinheit (1056) ferner Folgendes umfasst:
ein Speichermittel (1064), wobei das Speichermittel (1064) zum Speichern eines ersten Bildes ausgelegt ist, wobei das erste Bild während einer ersten Sitzung erfasst wurde, wobei das erste Bild eine erste Stellung des anatomischen Teils während der ersten Sitzung darstellt;
einen Mikroprozessor (1066), wobei der Mikroprozessor (1066) zum Empfangen des ersten Bilds von dem Speichermittel (1064) und des aktuellen Bilds von der Bildgebungsvorrichtung (1070) und zum Bestimmen, auf Basis des ersten Bilds und des aktuellen Bilds, eines wirksamen Drucks jeder der Fluidkammern (1008a-j) zur Anpassung der aktuellen Stellung des anatomischen Teils ausgelegt ist, so dass die aktuelle Stellung des anatomischen Teils im Wesentlichen der ersten Stellung des anatomischen Teils ähnelt; und
ein Signalisierungsmittel (1068), wobei das Signalisierungsmittel (1068) zum Empfangen von Anweisungen von dem Mikroprozessor (1066) bezüglich des wirksamen Drucks jeder der Fluidkammern (1008a-j) und zum Senden eines Anpassungssignals an den Druckregler (1004; 1006) ausgelegt ist, wobei das Anpassungssignal den Druckregler (1004; 1006) anweist, den Druck jeder der Fluidkammern (1008a-j) an den wirksamen Druck anzupassen.

10. System nach Anspruch 9, worin die Bildgebungsvorrichtung (1070) eine Videokamera ist und das aktuelle Bild ein bewegliches Bild ist.

## Revendications

1. Berceau (1000 ; 2000) pour placer une région anatomique d'un patient pendant un traitement par radiothérapie, le berceau (1000; 2000), le procédé comportant :
une pluralité de chambres gonflables (1008a-j ; 2010 ; 2012 ; 2014 ; 2016 ; 2018 ; 2020 ; 2022 ; 2024 ; 2026) à fluide, chacune des différentes chambres (1008a-j ; 2010 ; 2012 ; 2014 ; 2016 ; 2018 ; 2020 ; 2022 ; 2024 ; 2026) à fluide étant conçue pour exercer une force contre l'une de différentes surfaces de la région anatomique ; et
un régulateur de pression (1004 ; 1006 ; 2010 ; 2012 ; 2014 ; 2016 ; 2018 ; 2020 ; 2022 ; 2024 ; 2026) conçu pour régler la pression de chacune des chambres (1008a-j ; 2010 ; 2012 ; 2014 ; 2016 ; 2018 ; 2020 ; 2022 ; 2024 ; 2026) à fluide indépendamment de chaque autre chambre à fluide en envoyant du fluide dans ou en retirant du fluide de chacune des chambres (1008a-j ; 2010 ; 2012 ; 2014 ; 2016 ; 2018 ; 2020 ; 2022 ; 2024 ; 2026) à fluide ; **caractérisé en ce que**
au moins deux des différentes chambres (1008a-j ; 2010 ; 2012 ; 2014 ; 2016 ; 2018 ; 2020 ; 2022 ; 2024 ; 2026) à fluide sont conçues pour exercer des forces sensiblement opposées contre la région anatomique, de façon que la région anatomique soit immobilisée dans le berceau (1000 ; 2000).

2. Berceau (1000 ; 2000) selon la revendication 1, le berceau (1000 ; 2000) étant placé pour un patient en décubitus dorsal sur une couchette pendant le traitement.

3. Berceau (1000) selon la revendication 1, dans lequel la pluralité de chambres extensibles (1008a-j) à fluide comprennent :
une première chambre (1008c ; 1008d) à fluide conçue pour exercer une première force contre l'arrière du cou du patient ;
une deuxième chambre (1008e; 1008f) à fluide conçue pour exercer une deuxième force contre l'arrière de la tête du patient ;
une troisième chambre (1008i ; 1008j) à fluide conçue pour exercer une troisième force contre le côté gauche de la tête du patient ; et
une quatrième chambre (1008g ; 1008h) à fluide conçue pour exercer une quatrième force contre le côté droit de la tête du patient.

4. Berceau (1000) selon la revendication 3, dans lequel la première chambre (1008c ; 1008d) à fluide est conçue de façon que l'intensité de la première force augmente quand du fluide est introduit dans la première chambre (1008c ; 1008d) à fluide et que l'intensité diminue quand du fluide est retiré de la première chambre (1008c ; 1008d) à fluide.

5. Système pour placer une région anatomique d'un patient pendant un traitement par radiothérapie, le système comportant :
le berceau (1000 ; 2000) selon l'une quelconque des revendications précédentes,
et
une unité de commande (1056 ; 2056) conçue pour fournir au régulateur de pression (1004 ; 1006 ; 2010 ; 2012 ; 2014 ; 2016 ; 2018 ; 2020 ; 2022 ; 2024 ; 2026) des instructions afin de régler la pression de chacune des chambres (1008a-j ; 2010 ; 2012 ; 2014 ; 2016 ; 2018 ; 2020 ; 2022 ; 2024 ; 2026) à fluide de façon que la région anatomique soit maintenue dans une position voulue.

6. Système selon la revendication 5, dans lequel la position voulue est une position prédéterminée.

7. Système selon la revendication 6, dans lequel l''unité de commande (1056) comprend en outre :
un moyen formant mémoire (1064), le moyen formant mémoire (1064) étant conçu pour stocker des données de gonflage prédéterminées pour chacune des chambres (1008a-j) à fluide, les données de gonflage prédéterminées représentant une pression prédéterminée de chacune des chambres (1008a-j) à fluide, les pressions prédéterminées correspondant à une position prédéterminée de la région anatomique ;
un microprocesseur (1066), le microprocesseur (1066) étant conçu pour recevoir les données de gonflage prédéterminées issues du moyen formant mémoire (1064) et, d'après les données de gonflage prédéterminées, déterminer la pression prédéterminée de chacune des chambres (1008a-j) à fluide ; et
un moyen de transmission (1068) de signaux conçu pour recevoir du microprocesseur (1066) des instructions concernant la pression prédéterminée de chacune des chambres (1008a-j) à fluide et envoyer un signal de gonflage au régulateur (1004 ; 1006) de pression, le signal de gonflage demandant au régulateur (1004 ; 1006) de pression de régler à la pression prédéterminée la pression de chacune des chambres (1008a-j) à fluide.

8. Système selon la revendication 6, dans lequel la position prédéterminée de la région anatomique pendant une seconde séance de traitement est sensiblement similaire à une première position de la région anatomique pendant une première séance.

9. Système selon les revendications 5 et 8, comportant en outre :
un dispositif d'imagerie (1070), le dispositif d'imagerie (1070) étant conçu pour acquérir une image instantanée, l'image instantanée représentant une position instantanée de la région anatomique ;
l'unité de commande (1056) comprenant en outre :
un moyen formant mémoire (1064), le moyen formant mémoire (1064) étant conçu pour stocker une première image, la première image ayant été acquise pendant une première séance, la première image représentant une première position de la région anatomique pendant la première séance ;
un microprocesseur (1066), le microprocesseur (1066) étant conçu pour recevoir la première image du moyen formant mémoire (1064) et l'image instantanée du dispositif d'imagerie (1070) et, d'après la première image et l'image instantanée, déterminer une pression effective de chacune des chambres (1008a-j) afin de régler la position immédiate de la région anatomique, de façon que la position instantanée de la région anatomique soit sensiblement similaire à la première position de la région anatomique ; et
un moyen de transmission (1068) de signaux, le moyen de transmission (1068) de signaux étant conçu pour recevoir du microprocesseur (1066) une instruction concernant la pression effective de chacune des chambres (1008a-j) à fluide et envoyer un signal de réglage au régulateur (1004 ; 1006) de pression, le signal de réglage demandant au régulateur (1004 ; 1006) de pression de régler à la pression effective la pression de chacune des chambres (1008a-j) à fluide.

10. Système selon la revendication 9, dans lequel le dispositif d'imagerie (1070) est une caméra vidéo et l'image immédiate et une image animée.
